# EUROPEAN PATENT SPECIFICATION

(11) **EP 1 464 394 B1**
(45) Date of publication and mention of the grant of the patent: **05.09.2007**
(21) Application number: 03007285.4
(22) Date of filing: 31.03.2003
(51) Int. Cl.: B01J 23/44, B01J 37/16, B01J 37/02, C07B 37/04

(54) **Supported nanopalladium catalyst for C-C coupling reactions of haloarenes**
Geträgerter Nanopalladium Katalysator für C-C Kupplungsreaktionen von Haloarenen
Catalyseur à base de nanopalladium pour les réactions C-C de couplage d'haloarènes

(43) Date of publication of application: 06.10.2004
(73) Proprietor: COUNCIL OF SCIENTIFIC AND INDUSTRIAL RESEARCH, New Delhi 110 001 (IN)
(72) Inventor: Choudary, Boyapati, Manoranjan, Hyderabad-5, Andhra Pradesh (IN); Naidu, Madhi, Sateesh, Hyderabad-5, Andhra Pradesh (IN); Chowdari, Sreenivasa The Scripps Research Inst., La Jolla, CA 92037 (US); Kantam, Mannepalli, Lakshmi, Hyderabad-5, Andhra Pradesh (IN); Sreedhar, Bojja, Hyderabad-5, Andhra Pradesh (IN)
(74) Representative: Henkel, Feiler & Hänzel

(56) References cited:
- EP-A- 0 141 528
- WO-A-03/009936
- US-A- 4 482 752
- US-A- 5 976 486
- US-B1- 6 489 258
- NARAYANAN S ET AL: "Hydrotalcite-supported palladium catalysts - Part I: Preparation, characterization of hydrotalcites and palladium on uncalcined hydrotalcites for CO chemisorption and phenol hydrogenation" APPLIED CATALYSIS A: GENERAL, ELSEVIER SCIENCE, AMSTERDAM, NL, vol. 174, no. 1-2, 16 November 1998 (1998-11-16), pages 221-229, XP004271554 ISSN: 0926-860X

## Description

The present invention relates to a process for the preparation of a reusable heterogeneous nanopalladium(0) catalysts on the solid support to perfom C-C bond formation reactions such as Heck, Suzuki, Sonagashira, and Stille type reactions of haloarenes that include unreactive chloroarenes. Layered double hydroxides and Merrifield resin supported nanopalladium catalysts are prepared by an exchange of PdCl₄²⁻ followed by reduction and well characterized for the first time.

This invention particularly relates to an eco-friendly process employing re-usable heterogeneous catalyst in place of soluble palladium catalysts for preparing coupling products by C-C bond formation reaction of haloarenes that include unreactive chloroarenes in the presence of base. The ligand free heterogeneous layered double hydroxides supported nanopalladium (LDH-Pd⁰) catalyst using the basic LDH in place of basic ligands indeed exhibits higher activity and selectivity in the C-C coupling reactions of haloarenes that include electron poor and electron rich chloroarenes in nonaqueous ionic liquids (NAIL) over the homogeneous PdCl₂ system. The coupling products find good applications as intermediates in the preparation of materials, natural products, and bioactive compounds.

There are serious disadvantages in performing the catalytic C-C bond formation reaction with iodoarenes and bromoarcnes in the manufacture of olefins due to high cost of the starting materials. Despite the synthetic elegance and high turnover number, these coupling reactions suffer from serious limitations of using the expensive bromo and iodoarcnes that precluded the wide use in industry. By employing the heterogeneous catalytic system and chloroarenes as starting materials, the cost naturally comes down due to easy recovery of the catalyst and low cost of chloroarenes when compared with bromo and iodoarenes.

**References may be made** to the publications, Organometallics 1992, 11, 1995, Angew. Chem. Int. Ed. 1998, 37, 481, J. Organomet. Chem. 1998, 572, 93, Angew. Chem. Int. Ed. Engl. 1995, 34, 2371, J. Organomet. Chem. 1999, 576, 23 **wherein** Heck-olefination of chloroarenes is carried by palladium complexes under homogeneous conditions. **The inherent disadvantages** are difficulty in the recovery of palladium and usage of activated electron poor and nonactivatcd electron neutral chloroarenes.

**References may be made** to the publications Organometallics 1993, 12, 4734, J. Org. Chem. 1999, 64, 10, J. Am. Chem. Soc. 2001, 123,6989, Synlett, 2000, 11, 1589, J. Am. Chem. Soc. 1999, 121, 2123 **wherein** olefins are prepared from deactivated highly electron rich chloroarenes by palladium complexes. **The inherent disadvantage** is the usage of expensive sterically hindered phosphine palladium complexes.

**Reference may be made** to the publication Chem. Eur. J. 2000, 6, 1017 **wherein** Heck-olefination of deactivated highly electron rich chloroarenes is carried by palladacycle catalysts in the presence of nonaqueous liquids. **The inherent disadvantages** are the usage of additives and low yields.

**Reference may be made** to European patent DE-A 197 12 388.0,1997 **wherein** Heck-olefination of chloroarenes is carried by palladacycle catalyst using an additive. **The inherent disadvantage** is that no reaction occurs in the absence of phosphonium salt.

**Reference may be made** to the publications J. Am. Chem. Soc., 1998, 120, 9722 and Angew. Chem. Int. Ed, Engl., 1998, 37, 3387 **wherein** biphenyls arc prepared using arylchlorides with palladium catalysts. **The inherent disadvantage** is the usage of expensive, air-sensitive phosphine ligands.

**Reference may be made** to a publication J. Organometallic Chem., 1998, 557, 93 **wherein** biphenyls are prepared using carbene ancillary ligands with aryl bromides and activated aryl chlorides with palladium catalyst. **The drawbacks** are longer reaction times and yield from the aryl chloride was relatively low.

**Reference may be made** to a publication J. Organomet. Chem. 2001, 634, 39 wherein diarylacetylenes are prepared using heterogeneous polymer anchored bis(pyrimidine)-based palladium catalyst with chlorobenzene with impressive turnover frequency. **The drawback** is that it was not tried for electron rich chloroarenes.

**Reference may be made** to the publication Synlett, 2000, 1043 **wherein** arylstannanes were prepared using palladium catalyst in the presence of potassium acetate. **The drawback** is that the usage of aryl iodides as substrates.

### Objects of the invention

The main object of the present invention is to provide a process of forming C-C bonds in the presence of a ligand-free reusable heterogeneous nanopalladium(0) catalyst.

### Summary of the Invention

The present invention provides a process of forming C-C bonds, said process comprising the step of reacting haloarenes in a Heck, Suzuki, Sonagashira, or Stille type reaction optionally under microwave irradiation using solvents selected from nonaqueous ionic liquid, THF, dioxane, water, NMP at a temperature ranging between 20 to 150°C for a period ranging from 0.5 to 48 h under nitrogen atmosphere in the presence of a ligand-free reusable heterogeneous nanopalladium (0) catalyst of the formula [M(II)₁₋ₓM(III)ₓ(OH)₂] [Pd(0)] obtained from layered double hydroxides (LDH) of the formula [M(II)₁₋ₓM(III)ₓ(OH)₂] wherein LDH is a class of layered material consisting of alternating cationic M(II)₁₋ₓM(III)ₓ(OH)₂^{x+} and anionic Aⁿ⁻·zH₂O layers, M^{II} is a divalent cation selected from the group consisting of Mg²⁺, Mn²⁺, Fe²⁺, CO²⁺, Ni²⁺, Cu²⁺, Zn²⁺ and Ca²⁺ and M^{III} is a trivalent ion selected from the group consisting of Al³⁺, Cr³⁺, Mn³⁺, Fe³⁺ and CO³⁺, Aⁿ⁻ is an interstitial anion selected from nitrate, carbonate and chloride, x is the mole fraction having integral value ranging from 0.2 to 0.33, and z is the number of water molecules and ranges from 1 to 4, by an exchange of Aⁿ⁻ ions with an aqueous solution of Na₂PdC₄, followed by a reduction on the LDH support.

**In an embodiment of the present invention,** the quantity of nanopalladium(0) catalyst used in the reaction is 0.1 to 3 mol% of palladium with respect to the substrate.

**In an embodiment of the present invention,** nanopalladium (0) catalysts are recovered by simple filtration and reused for several cycles with consistent activity.

**In another embodiment of the present invention,** the solvents selected for the C-C bond formation reactions are nonaqueous ionic liquid, water, 1,4-dioxane, THF, NMP or any mixture thereof.

**In yet another embodiment of the present invention,** the base used is selected from the known group consisting of triethylamine, tributylamine, potassium fluoride, potassium acetate, and obtaining the desired product by chromatography or recrystallization.

**In still another embodiment of the present invention,** is to reduce the reaction time by employing microwave irradiation. Using microwave irradiation, the rate of C-C coupling reaction is accelerated manifold with the highest turnover frequency ever recorded both in the case of electron poor and electron rich chloroarenes.

**In yet another embodiment of the present invention,** a phosphine free new recyclable heterogeneous catalytic system was developed to dispense the use of expensive and air sensitive basic phosphines for palladium catalyzed coupling reactions of chloroarenes. The basic support, Mg-Al layered double hydroxides (LDH) selected not only stabilizes the nanopalladium particles but also provides the adequate electron density to the anchored Pd⁰ species to facilitate oxidative addition of even the deactivated electron rich chloroarenes.

### Detailed Description of the Invention:

Heterogeneous nanopalladium(0) catalysts are prepared by an exchange of PdCl₄²⁻ on the support selected from LDH in an aqueous solvent at a temperature ranging between 20 to 30 °C for a period ranging from 5 to 24 h under nitrogen atmosphere followed by filtration and reduction with hydrazine hydrate in ethanol at room temperature. Filtration gave the desired nanopalladium(0) catalysts.

C-C bond coupling products are prepared by using the recyclable nanopalladium(0) catalysts by Heck, Suzuki, Sonagashira, and Stille type reactions of haloarenes that include unreactive chloroarenes in a under standard thermal and microwave conditions using solvents selected from nonaqueous ionic liquid, water, THF, dioxan, and NMP at a temperature ranging between 20 to 150 °C for a period 0.5 to 48 h under nitrogen atmosphere, and obtaining the pure C-C coupling products by a conventional method.

The palladium content in the catalyst ranges between 0.1 to 3 mol% with respect to the substrate. Nanopalladium (0) catalysts are recovered by simple filtration and reused for several cycles with consistent activity.

The solvents selected for the C-C bond formation reaction is selected from the group consisting of nonaqueous ionic liquid, water, 1,4-dioxane, THF, NMP or any mixture thereof. The base used is selected from the group consisting of triethylamine, tributylamine, potassium fluoride, potassium acetate.

### Scientific Explanation:

In the present invention, we synthesized LDH supported nanopalladium catalysts for the first time and used in catalytic amounts for preparing coupling products by C-C bond formation reactions involving Heck-, Suzuki-, Sonogashira- and Stille type coupling of haloarenes that include chloroarenes in presence of base.

Heterogeneous nanopalladium(0) catalysts are prepared by an exchange of PdCl₄²⁻ followed by reduction on LDH.

The nanopalladium (0) on supports is responsible for the activity of catalyst in C-C coupling reactions. The activity of heterogeneous nanopalladium catalysts is similar or higher than the homogeneous counter parts. The basic support, Mg-Al layered double hydroxides (LDH) is selected as the material of choice, which not only stabilizes the nanopalladium particles but also provides the adequate electron density to the anchored Pd⁰ species to facilitate oxidative addition of even the deactivated electron rich chloroarenes.

Higher yields and stereoselectivities are obtained when nanopalladium catalysists are used. Incidentally this forms the first report of heterogeneous palladium catalyst employed in the Stille type coupling. The consistent activity obtained for several cycles makes the process economical and possible for commercial realization.

The coupling products find good applications as intermediates in the preparation of materials, natural products, and bioactive compounds. Thus this invention offers the best techno-economic route for the synthesis of coupling products. Therefore, nanopalladium catalysts are better option for the C-C bond formation reaction of haloarenes that include chloroarenes.

Nanopalladium catalysts are prepared as exemplified and used in catalytic amounts for preparing coupling products by C-C bond formation reaction in presence of base in a heterogeneous way as described in the examples.
The following examples arc given by way of illustration of the present invention and therefore should not be construed to limit the scope of the invention.

### Preparation of Nanopalladium catalysts

### Example 1

### Preparation of LDH (Mg-Al-Cl) (1)

A mixture of MgCl₂.6H₂O (30.49 g, 0.15 mmol) and AlCl₃.6H₂O (12.07 g, 0.05 mmol) was dissolved in 200 ml of deionised water. To this aqueous solution, 100 ml of NaOH (2M) solution was slowly added at 25°C and a further amount of 2M NaOH solution was added to maintain a pH of 10 under nitrogen flow. The resulting suspension was stirred overnight at 70°C. The solid product was isolated by filtration, washed thoroughly with deionised water and dried overnight at 80°C. All the synthetic steps were carried out using decarbonated water.

### Example 2

### Preparation of Na₂PdCl₄ (2)

Na₂PdCl₄ was prepared by refluxing PdCl₂ (1.77 g, 10 mmol) and sodium chloride (0.58 g, 10 mmol) in 50 mL for 4 h. The solution was filtered in hot condition to avoid NaCl contamination. Evaporation of the filtrate gave dark brown flakes (2.88 g, 98%).

### Example 3

### Preparation of LDH-PdCl₄ (3)

Mg-Al-Cl (1.5 g) was suspended in 150 mL of aqueous Na₂PdCl₄ (0.441 g, 1.5 mmol) solution and stirred at 25°C for 12 h under nitrogen atmosphere. The solid catalyst was filtered, washed thoroughly with 500 mL of water and vacuum-dried to obtain 1.752 g of LDH-PdCl₄ (0.86 mmol of Pd per gram).

### Example 4

### Preparation of LDH-Pd⁰ (4)

LDH-PdCl₄ (1 g) was reduced with hydrazine hydrate (1 g, 20 mmol) in ethanol (10 mL) for 3 h at room temperature, filtered and washed with ethanol to give an air stable black powder (0.95 mmol of Pd per gram).

### C-C Bond forming reactions

The C-C bond forming reactions were performed using LDH-Pd⁰ catalysts to evaluate nanopalladium catalysts of the present invention.

### 1. Heck-Olefination.

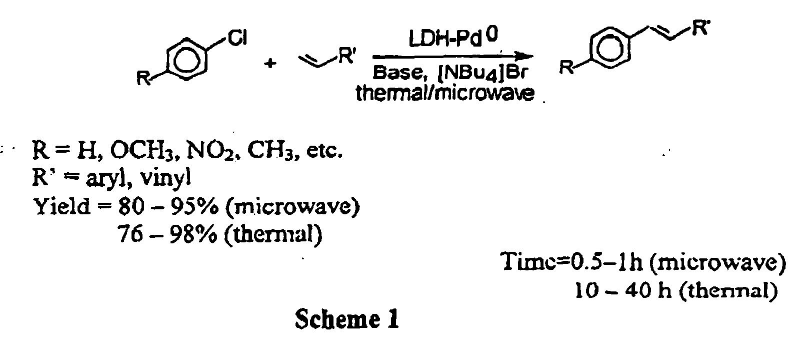

### Example 9

### Heck-Olefination between chlorobenzene and styrene catalysed by LDH-Pd⁰ under thermal conditions

In a 100 mL Schlenk flask, the NAIL (3.23 g, 10 mmol)(NBu₄Br) was heated to melt (130°C) and degassed with nitrogen and vacuum prior to the addition of other reagents. After cooling the NAIL to room temperature, LDH-Pd⁰ (3 mol%), tri-n-butyl amine (222mg, 1.2 mmol) were added. The styrene (1.2 mmol) and chlorobenzene (1 mmol) were then added and the reaction was heated to 130°C and stirred for 10-40 h under nitrogen atmosphere. After completion of the reaction, the LDH-Pd⁰ catalyst was filtered and washed with water and dichloromcthane. After removing the solvent, the crude material was chromatographed on silica gel or recrystallized from ethanol to afford the *trans*-stilbene

### Examples 10-17

The procedure was followed as in example 9 with various substrates under thermal conditions and the results are presented in Table 1.

**Table 1. LDH-Pd⁰ Catalyzed Heck-Olefination of Chloroarenes under thermal conditions^{a}**

| Example | Chloroarene | Olefin | Time (h) | Yield (%) |
|---|---|---|---|---|
| 9 | 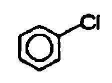 | 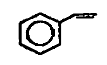 | 30 | 98 |
| 10 | 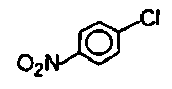 | 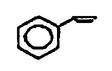 | 10 | 95 |
| 11 | 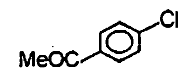 | 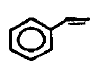 | 15 | 97 |
| 12 | 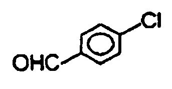 | 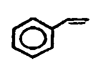 | 15 | 98 |
| 13 | 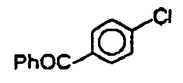 | 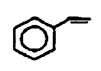 | 15 | 93 |
| 14 | 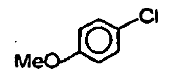 | 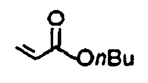 | 40 | 86 |
| 15 | 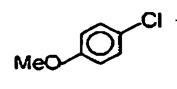 | 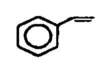 | 40 | 76(5)*^{b}* |
| 16 | 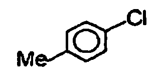 | 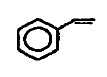 | 30 | 96(55)*^{b}* |
| 17 | 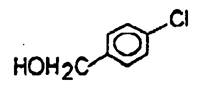 | 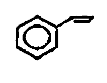 | 30 | 92(51)*^{b}* |

| | | | | |
|---|---|---|---|---|
| *^{a}* Chloroarene (1 mmol), olefin (1.2 mmol) LDH-Pd⁰ (3 mol%), [NBu₄]Br (10 mmol), and tri-*n*-butyl amine (1.2 mmol). Reactions are conducted at 130°C. *^{b}* The values in parentheses refer to the homogeneous reaction conducted with PdCl₂ under identical conditions. | | | | |

### Example 18

### Heck-Olefination between chlorobenzene and styrene catalysed by LDH-Pd⁰ under microwave conditions.

Styrene (1.2 mmol), chlorobenzene (1 mmol), LDH-Pd⁰ (3 mol%), tri-n-buityl amine (222mg, 1.2 mmol) and NAIL (3.23 g, 10 mmol) were taken in a teflon vessel, closed and irradiated in a Miele Electronic M270 microwave oven at 400W and 130°C for 0.5-1 h. After completion of the reaction, the LDH-Pd⁰ catalyst was filtered and washed with water and dichloromethane. After removing the solvent, the crude material was chromatographed on silica gel to afford the *trans*-stilbene.

### Examples 19-26

The procedure was followed as in example 18 with various substrates under microwave conditions and the results are presented in Table 2.

**Table 2. LDH-Pd⁰ Catalyzed Heck-Olefination of Chloroarenes under microwave conditions^{a}**

| Example | Chloroarene | Olefin | Time (h) | Yield (%) |
|---|---|---|---|---|
| 18 | 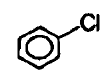 | 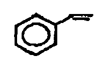 | 0.5 | 95 |
| 19 | 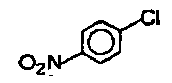 | 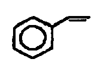 | 0.5 | 96 |
| 20 | 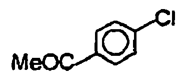 | 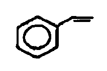 | 0.5 | 93 |
| 21 | 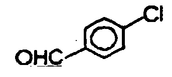 | 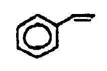 | 0.5 | 95 |
| 22 | 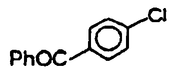 | 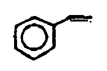 | 0.5 | 91 |
| 23 | 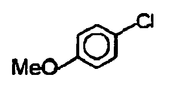 | 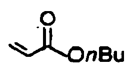 | 1 | 85 |
| 24 | 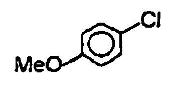 | 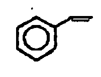 | 1 | 80 |
| 25 | 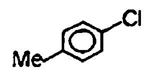 | 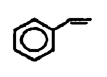 | 0.5 | 92 |
| 26 | 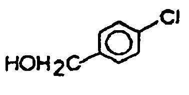 | 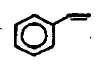 | 0.5 | 90 |

| | | | | |
|---|---|---|---|---|
| *^{a}* Chloroarcne (1 mmol), olefin (1.2 mmol) LDH-Pd⁰ (3 mol%), [NB_{LL4}]Br (10 mmol), and tri-*n*-butyl amine (1.2 mmol). Microwave power 400W and temperature 130°C. | | | | |

### Examples 27-32

In an effort to compare the reactivity of LDH-Pd⁰ with other heterogeneous catalysts namely Pd/C, Pd/SiO₂, resin-Pd⁰ and Pd/Al₂O₃ in the Heck-olefination, separate experiments were conducted under identical conditions with the same ingredients and the results are summarized in Table 3. The activity of various catalysts in the Heck-olefination of 4-chloroanisole is found to be in the order: LDH-Pd⁰>resin-Pd⁰>Pd/C>Pd/Al₂O₃>Pd/SiO₂. These results indicate that the basic support, LDH facilitates the oxidative addition of Pd° with 4-chloroanisole and eventually the Heck-olefination reaction.

The procedure was followed as in example 9 with various catalysts between 4-chloroanisole and styrene under thermal conditions and the results are presented in Table 3.

**Table 3. Heck-Olefination of 4-Chloroanisole with Styrene Using Various Palladium Catalysts^{a}**

| Example | Catalyst | Yield (%) |
|---|---|---|
| 27 | LDH-Pd⁰ | 76 |
| 28 | resin-Pd⁰ | 29 |
| 29 | Pd/C | 28 |
| 30 | Pd/Al₂O₃ | 22 |
| 31 | Pd/SiO₂ | 15 |
| 32 | PdCl₂ | 5 |

| | | |
|---|---|---|
| *^{a}* 4-chloroanisole (1 mmol), olefin (1.2 mmol). Palladium catalyst (3 mol%), [NBu₄]Br 3.23 g (10 mmol), and tributylamine (1.2 mmol) were stirred at 130°C for 40 h. | | |

### 2. Suzuki coupling

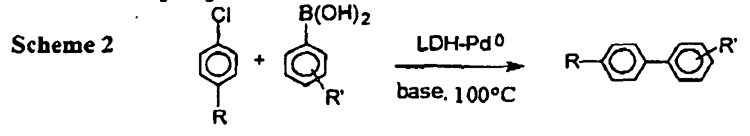

### Example 33

### Suzuki coupling between chlorobenzene and phenylboronic acid catalysed by LDH-Pd⁰

Chlorobenzene (1 mmol), phenylboronic acid (1.5 mmol), potassium fluoride (3 mmol), LDH-Pd⁰ (1 mol%) and 1,4-dioxane-water (5:1, 5 mL) were charged in a round-bottomed flask. Reactions were carried out at 100°C for 10 h. After completion of the reaction (monitored by TLC), the catalyst was filtered and the reaction mixture was poured into water and the aqueous phase was extracted with ether. After drying, the corresponding product was purified by crystallization from diethyl ether-pentane.

### Examples 34-40

The procedure was followed as in example 33 and the results are given in Table 4.

**Table 4. Suzuki Coupling Reactions of Chloroarenes with Arylboronic Acids^{a}**

| Example | Arylboronic acid | Haloarene | Yield(%) |
|---|---|---|---|
| 33 | 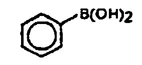 | 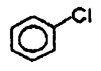 | 93(92)*^{b}* |
| 34 | 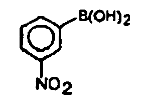 | 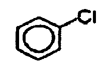 | 90 |
| 35 | 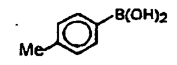 | 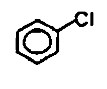 | 60 |
| 36 | 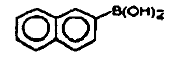 | 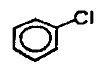 | 80 |
| 37 | 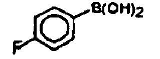 | 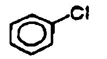 | 70 |
| 38 | 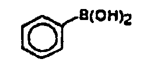 | 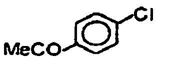 | 60 |
| 39 | 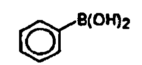 | 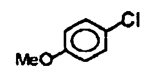 | 90 |
| 40 | 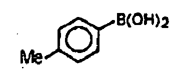 | 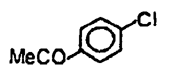 | 88 |

| | | | |
|---|---|---|---|
| *^{a}* Chloroarene (1 mmol), arylboronic acid (1.5 mmol), LDH-Pd⁰ (1 mol%) and KF (3 mmol), 100°C, 10 h. All the reactions were carried out with 1,4-dioxane-water (5:1, 5 mL) as solvent. *^{b}* Under NAIL conditions, 8 h. | | | |

### 3. Sonogashira Coupling

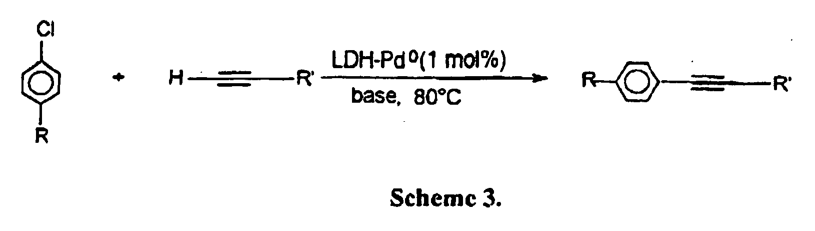

### Example 41

### Sonogashira coupling between chlorobenzene and phenylacetylene catalysed by LDH-Pd⁰

To a stirred slurry of chlorobenzene (1 mmol), cuprous iodide (7.6mg, 0.04 mmol), and LDH-Pd⁰ (1 mol%), in THF (2 mL) and water (4 mL) was added triethylamine (152 mg, 1.5 mmol). A solution of 1.25 mmol of phenylacetylene in THF (2 mL) was then added over 2 h. After completion of the reaction, the solvent was evaporated, and the residue was treated with pentane. The solution was filtered to obtain the catalyst and evaporation of the solvent gives the coupling product.

### Examples 42-43

The procedure was followed as in example 41 and the results are given in Table 5.

**Table 5. Cross Coupling Reactions Between Phenylacetylene and Chloroarene**

| Example | Chloroarene | Phenylacetylene | Time (h) | Yields (%) |
|---|---|---|---|---|
| 41 | 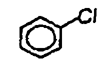 | 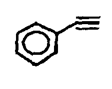 | 30 | 95 |
| | | | 27 | 95*^{b}* |
| 42 | 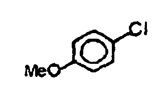 | 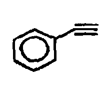 | 48 | 60 |
| 43 | 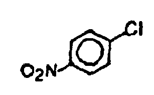 | 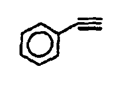 | 40 | 82 |

| | | | | |
|---|---|---|---|---|
| *^{a}* Chloroarene (1 mmol), phenylacetylene (1.1 mmol), LDH-Pd⁰ (1 mol%), Et₃N (1.5 mol), 80°C. All reactions were carried out with THF-water (1:1, 8 mL) as solvent system. *^{b}* Under NAIL conditions. | | | | |

### 4. Stille type coupling

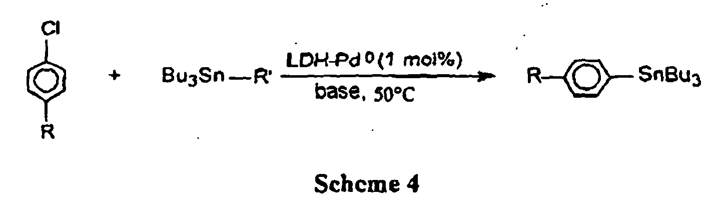

### Example 44

### Stille type couping between chlorobenzene and tributyltin reagent catalysed by LDH-Pd⁰

A round-bottomed flask was charged with LDH-Pd⁰ (1 mol%), potassium acetate (294mg, 3 mmol), and NMP (4 ml). To this 4-chloroanisole (1 mmol) and tributyltin hydride (2 mmol) were added successively, and the mixture was stirred at 50⁰ C for 16 h. The reaction mixture was diluted with benzene, washed with water and catalyst was collected for next cycle.

### Examples 45-49

The procedure was followed as in example 44 and the results are given in Table 6.

**Table 6. Stannylation of Haloarenes with LDH-Pd⁰ catalyst^{a}**

| Example | Chloroarene | Tin reagent | Yield |
|---|---|---|---|
| 44 | 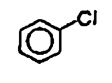 | Bu₃Sn-H | 90(91)*^{b}* |
| 45 | 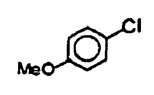 | Bu₃Sn-H | 80 |
| 46 | 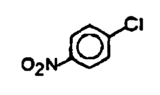 | Bu₃Sn-H | 90 |
| 47 | 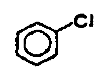 | 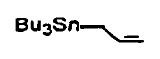 | 80 |
| 48 | 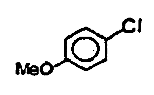 | 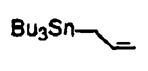 | 65 |
| 49 | 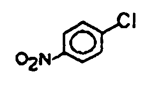 | 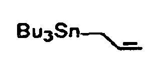 | 70 |

| | | | |
|---|---|---|---|
| *^{a}* Chloroarene (1 mmol), trialkyltin reagent (2 mmol), potassium acetate (3mmol), LDH-Pd⁰ (2 mol%). All the reactions were carried out with NMP as solvent, 50°C, 16 h. *^{b}* Under NAIL conditions, 12 h. | | | |

The main advantages of the present invention are:
1. A novel and ecofriendly process for the synthesis of coupling products from haloarenes that include highly unreactive chloroarenes by C-C bond formation is presented.
2. The present process dispenses the use of soluble palladium catalysts instead a heterogeneous reusable LDH-Pd(0) is used.
3. Nanopalladium catalyst, LDH-Pd(0) is prepared and used as heterogeneous catalyst for the synthesis of coupling products by C-C bond formation reactions. The use of heterogeneous nanopalladium catalyst precludes the presence of palladium in traces with the product.
4. The stereoselectivity and the yields are good.
5. The work-up procedure is simple.
6. The catalyst is subjected to many recycles, which displayed consistent activity.
7. The present process is environmentally safe since there is no disposal problem.
8. The process is economical.

## Claims

1. A process of forming C-C bonds, said process comprising the step of reacting haloarenes in a Heck, Suzuki, Sonagashira or Stille type reaction optionally under microwave irradiation using solvents selected from nonaqueous ionic liquid, THF, dioxane, water, NMP at a temperature ranging between 20 to 150 °C for a period ranging from 0.5 to 48 h under nitrogen atmosphere in the presence of a ligand-free reusable heterogeneous nanopalladium (0) catalyst of the formula [M(II)₁₋ₓM(III)ₓ(OH)₂] [Pd(0)] obtained from layered double hydroxides (LDH) of the formula [M(II)₁₋ₓM(III)ₓ(OH)₂^{x+}] [Aⁿ⁻] wherein LDH is a class of layered material consisting of alternating cationic M(II)₁₋ₓM(III)ₓ(OH)₂^{x+} and anionic Aⁿ⁻·zH₂O layers, M^{II} is a divalent cation selected from the group consisting of Mg²⁺, Mn²⁺, Fe²⁺, Co²⁺, Ni²⁺, Cu²⁺, Zn²⁺ and Ca²⁺ and M^{III} is a trivalent ion selected from the group consisting of Al³⁺, Cr³⁺, Mn³⁺, Fe³⁺ and CO³⁺, Aⁿ⁻ is an interstitial anion selected from nitrate, carbonate and chloride, x is the mole fraction having integral value ranging from 0.2 to 0.33, and z is the number of water molecules and ranges from 1 to 4, by an exchange of Aⁿ⁻ ions with an aqueous solution of Na₂PdCl₄, followed by a reduction on the LDH support.

2. A process as claimed in claim 1, wherein said catalyst LDH-Pd⁰ of the formula [M(II)₁₋ₓM(III)ₓ(OH)₂] [Pd(0)] is obtained by reacting sodium palladate of formula Na₂PdCl₄ with an LDH of formula [M(II)₁₋ₓM(III)ₓ(OH)₂^{x+}] [Aⁿ⁻] wherein M^{II} is a divalent cation selected from the group consisting of Mg²⁺, Mn²⁺, Fe²⁺, CO²⁺, Ni²⁺, Cu²⁺, Zn²⁺ and Ca²⁺ and M^{III} is a trivalent ion selected from the group consisting of Al³⁺, Cr³⁺, Mn³⁺, Fe³⁺ and CO³⁺, and Aⁿ⁻ is an interstitial anion selected from nitrate, carbonate and chloride, x is the mole fraction having integral value ranging from 0.2 to 0.33, and z is the number of water molecules and ranges from 1 to 4, in an aqueous solvent at a temperature ranging between 20 to 30° C for a period of 5 to 24 h under the nitrogen atmosphere followed by reduction, filtration and washing to obtain the desired catalyst.

3. A process as claimed in claims 1 or 2, wherein the palladium content in the catalyst ranges between 0.1 to 3 mol% with respect to the substrate.

4. A process as claimed in any one of claims 1 to 3, wherein nanopalladium (0) catalysts are recovered by simple filtration and reused for several cycles with consistent activity.

5. A process as claimed in any one of claims 1 to 4, wherein the solvents for the C-C bond formation reaction are selected from the group consisting of nonaqueous ionic liquid, water, dioxane, THF, NMP or any mixture thereof.

6. A process as claimed in any one of claims 1 to 5, wherein the base used in said reaction of forming C-C bonds, is selected from the group consisting of triethylamine, tributylamine, potassium fluoride, potassium acetate and obtaining the desired product by chromatography or recrystallization.

## Patentansprüche

1. Verfahren zur Bildung von C-C-Bindungen, wobei das Verfahren die Stufe der Umsetzung von Halogenarenen in einer Reaktion des Heck-, Suzuki-, Sonagashira- oder Stilletyps, optional unter Mikrowellenbestrahlung, unter Verwendung von Lösemitteln, die aus einer nichtwässrigen ionischen Flüssigkeit, THF, Dioxan, Wasser, NMP ausgewählt sind, bei einer Temperatur im Bereich zwischen 20 und 150 °C über einen Zeitraum, der im Bereich von 0,5 bis 48 h liegt, in Stickstoffatmosphäre in Gegenwart eines ligandfreien wieder verwendbaren heterogenen Nanopalladium(0)-Katalysators der Formel [M(II)₁₋ₓM(III)ₓ(OH)₂] [Pd(0)] umfasst, wobei der Katalysator ausgehend von schichtförmigen Doppelhydroxiden (LDH) der Formel [M(II)₁₋ₓM(III)ₓ(OH)₂] [Aⁿ⁻], wobei LDH eine Klasse eines schichtförmigen Materials ist, das aus alternierenden kationischen M(II)₁₋ₓM(III)ₓ(OH)₂^{x+}- und anionischen Aⁿ⁻·zH₂O-Schichten besteht, M^{II} für ein zweiwertiges Kation, das aus der Gruppe von Mg²⁺, Mn²⁺, Fe²⁺, CO²⁺, Ni²⁺, Cu²⁺, Zn²⁺ und Ca²⁺ ausgewählt ist, steht und M^{III} für ein dreiwertiges Ion, das aus der Gruppe von Al³⁺, Cr³⁺, Mn³⁺, Fe³⁺ und CO³⁺ ausgewählt ist, steht, Aⁿ⁻ für ein interstitielles Anion, das aus Nitrat, Carbonat und Chlorid ausgewählt ist, steht, x der Molenbruch mit einem Integralwert im Bereich von 0,2 bis 0,33 ist und z die Zahl der Wassermoleküle ist und im Bereich von 1 bis 4 liegt, durch Austausch von Aⁿ⁻-Ionen mit einer wässrigen Lösung von Na₂PdCl₄ und anschließende Reduktion auf dem LDH-Träger erhalten wird.

2. Verfahren nach Anspruch 1, wobei der Katalysator LDH-Pd° der Formel [M(II)₁₋ₓM(III)ₓ(OH)₂] [Pd(0)] durch Umsetzung von Natriumpalladat der Formel Na₂PdCl₄ mit einem LDH der Formel [M(II)₁₋ₓM(III)ₓ(OH)₂^{x+}] [Aⁿ⁻], wobei M^{II} für ein zweiwertiges Kation, das aus der Gruppe von Mg²⁺, Mn²⁺, Fe²⁺, Co²⁺, Ni²⁺, Cu²⁺, Zn²⁺ und Ca²⁺ ausgewählt ist, steht und M^{III} für ein dreiwertiges Ion, das aus der Gruppe von Al³⁺, Cr³⁺, Mn³⁺, Fe³⁺ und Co³⁺ ausgewählt ist, steht und Aⁿ⁻ für ein interstitielles Anion, das aus Nitrat, Carbonat und Chlorid ausgewählt ist, steht, x der Molenbruch mit einem Integralwert im Bereich von 0,2 bis 0,33 ist und z die Zahl der Wassermoleküle ist und im Bereich von 1 bis 4 liegt, in einem wässrigen Lösemittel bei einer Temperatur im Bereich zwischen 20 und 30°C über einen Zeitraum von 5 bis 24 h in Stickstoffatmosphäre, anschließende Reduktion, Filtration und Waschen unter Bildung des gewünschten Katalysators erhalten wird.

3. Verfahren nach Anspruch 1 oder 2, wobei der Palladiumgehalt in dem Katalysator im Bereich zwischen 0,1 und 3 Mol-% in Bezug auf das Substrat liegt.

4. Verfahren nach einem der Ansprüche 1 bis 3, wobei Nanopalladium(0)katalysatoren über mehrere Zyklen mit konsistenter Aktivität durch einfache Filtration rückgewonnen und wiederverwendet werden.

5. Verfahren nach einem der Ansprüche 1 bis 4, wobei die Lösemittel für die C-C-Bindungsbildungsreaktion aus der Gruppe von einer nichtwässrigen ionischen Flüssigkeit, Wasser, Dioxan, THF, NMP oder einem Gemisch derselben ausgewählt sind.

6. Verfahren nach einem der Ansprüche 1 bis 5, wobei die bei der Reaktion der Bildung von C-C-Bindungen verwendete Base aus der Gruppe von Triethylamin, Tributylamin, Kaliumfluorid, Kaliumacetat ausgewählt ist und das gewünschte Produkt durch Chromatographie oder Umkristallisation erhalten wird.

## Revendications

1. Procédé pour former des liaisons C-C, ledit procédé comprenant l'étape consistant à faire réagir des halogénoarènes dans une réaction de type Heck, Suzuki, Sonagashira ou Stille, facultativement sous irradiation de micro-ondes en utilisant des solvants choisis parmi un liquide ionique non aqueux, le THF, le dioxane, l'eau, la NMP à une température allant de 20 à 150°C pendant une période allant de 0,5 à 48 h sous atmosphère d'azote en présence d'un catalyseur à nanopalladium (0) hétérogène réutilisable exempt de ligand, de formule [M(II)₁₋ₓM(III)ₓ(OH)₂] [Pd(0)] obtenu à partir d'hydroxydes doubles stratifiés (LDH) de formule [M(II)₁₋ₓM(III)ₓ(OH)₂^{x+}] [Aⁿ⁻] où LDH est une classe de matériau stratifié consistant en des couches cationique M(II)₁₋ₓM(III)ₓ(OH)₂^{x+} et anionique Aⁿ⁻·zH₂O alternées, M^{II} est un cation divalent choisi dans le groupe constitué par Mg²⁺, Mn²⁺, Fe²⁺, Co²⁺, Ni²⁺, Cu²⁺, Zn²⁺ et Ca²⁺ et M^{III} est un ion trivalent choisi dans le groupe constitué par Al³⁺, Cr³⁺, Mn³⁺, Fe³⁺ et Co³⁺, Aⁿ⁻ est un anion interstitiel choisi parmi un nitrate, un carbonate et un chlorure, x est la fraction molaire ayant une valeur intégrale allant de 0,2 à 0,33, et z est le nombre de molécules d'eau et va de 1 à 4, par un échange d'ions Aⁿ⁻ avec une solution aqueuse de Na₂PdCl₄ suivie par une réduction sur le support LDH.

2. Procédé selon la revendication 1, dans lequel ledit catalyseur LDH-Pd⁰ de formule [M(II)₁₋ₓM(III)ₓ (OH)₂] [Pd(0)] est obtenu en faisant réagir du palladate de sodium de formule Na₂PdCl₄ avec un LDH de formule [M(II)₁₋ₓM(III)ₓ(OH)₂^{x+}] [Aⁿ⁻] dans laquelle M^{II} est un cation divalent choisi dans le groupe constitué par Mg²⁺, Mn²⁺, Fe²⁺, Co²⁺, Ni²⁺, Cu²⁺, Zn²⁺ et Ca²⁺ et M^{III} est un ion trivalent choisi dans le groupe constitué par Al³⁺, Cr³⁺, Mn³⁺, Fe³⁺ et Co³⁺, et Aⁿ⁻ est un anion interstitiel choisi parmi un nitrate, un carbonate et un chlorure, x est la fraction molaire ayant une valeur intégrale allant de 0,2 à 0,33, et z est le nombre de molécules d'eau et va de 1 à 4, dans un solvant aqueux à une température allant de 20 à 30°C pendant une période de 5 à 24 h sous l'atmosphère d'azote puis en réduisant, en filtrant et en lavant pour obtenir le catalyseur souhaité.

3. Procédé selon la revendication 1 ou 2, dans lequel la teneur en palladium dans le catalyseur va de 0,1 à 3 % en moles par rapport au substrat.

4. Procédé selon l'une quelconque des revendications 1 à 3, dans lequel les catalyseurs à nanopalladium (0) sont recueillis par simple filtration et réutilisés sur plusieurs cycles avec une activité constante.

5. Procédé selon l'une quelconque des revendications 1 à 4, dans lequel les solvants pour la réaction de formation de liaison C-C sont choisis dans le groupe constitué par un liquide ionique non aqueux, l'eau, le dioxane, le THF, la NMP ou tout mélange de ceux-ci.

6. Procédé selon l'une quelconque des revendications 1 à 5, dans lequel la base utilisée dans ladite réaction de formation de liaisons C-C est choisie dans le groupe constitué par la triéthylamine, la tributylamine, le fluorure de potassium, l'acétate de potassium et le produit souhaité est obtenu par chromatographie ou recristallisation.
